(19) Europäisches Patentamt
European Patent Office
Office européen des brevets

(11) Veröffentlichungsnummer: **0 001 399**
**B1**

(12) **EUROPÄISCHE PATENTSCHRIFT**

(45) Veröffentlichungstag der Patentschrift: **10.11.82**

(21) Anmeldenummer: **78100841.2**

(22) Anmeldetag: **07.09.78**

(51) Int. Cl.³: **C 07 D 213/30,**
A 01 N 43/40

(54) **Phenoxy-pyridinyl-alkanole, Verfahren zu ihrer Herstellung sowie ihre Verwendung als Fungizide.**

(30) Priorität: **20.09.77 DE 2742173**

(43) Veröffentlichungstag der Anmeldung:
**18.04.79 Patentblatt 79/8**

(45) Bekanntmachung des Hinweises auf die Patenterteilung:
**10.11.82 Patentblatt 82/45**

(84) Benannte Vertragsstaaten:
**BE CH DE FR GB NL**

(56) Entgegenhaltungen:
**US - A - 3 869 456**
**US - A - 4 043 790**
Die Akte enthält technische Angaben, die nach dem Eingang der Anmeldung eingereicht wurden und die nicht in dieser Patentschrift enthalten sind.

(73) Patentinhaber: **BAYER AG**
**Zentralbereich Patente, Marken und Lizenzen**
**D-5090 Leverkusen 1, Bayerwerk (DE)**

(72) Erfinder: **Holmwood, Graham, Dr.**
**Katernberger Strasse 184**
**D-5600 Wuppertal 1 (DE)**
Erfinder: **Frohberger, Paul-Ernst, Dr.**
**Willi-Baumeister-Strasse 5**
**D-5090 Leverkusen 1 (DE)**
Erfinder: **Brandes, Wilhelm, Dr.**
**Eichendorffstrasse 3**
**D-5653 Leichlingen (DE)**
Erfinder: **Paul, Volker, Dr.**
**Ahornstrasse 5**
**D-5650 Solingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 399 B1

Phenoxy-pyridinyl-alkanole, Verfahren zu ihrer Herstellung sowie ihre Verwendung als Fungizide

Die Erfindung betrifft neue Phenoxy-pyridinyl-alkanole, ein Verfahren zu ihrer Herstellung sowie ihre Verwendung also Fungizide auf dem Pflanzensektor.

Es ist bereits bekannt geworden, daß Trityl-1,2,4-triazole, wie Triphenyl-(1,2,4-triazol-1-yl)-methan, eine gute fungizide Wirksamkeit besitzen (vgl. DT—OS 1 795 249). Deren Wirkung ist jedoch, insbesondere bei niedrigen Aufwandmengen und -konzentrationen, nicht immer ganz befriedigend. Weiterhin ist allgemein seit längerer Zeit bekannt, daß Zink-ethylen-1,2-bisdithiocarbamidat ein gutes Mittel zur Bekämpfung von pilzlichen Pflanzenkrankheiten ist [vgl. Phytopathology *33*, 1113 (1963)]. Jedoch ist dessen Einsatz als Saatgutbeizmittel nur beschränkt möglich, da es bei niedrigen Aufwandmengen und -konzentrationen wenig wirksam ist.

Ferner ist bereits bekannt, daß bestimmte 5-Pyrimidincarbinole zur Bekämpfung von phytopathogenen Pilzen geeignet sind (vg. US—PS 3 869 456). Die Wirksamkeit dieser Stoffe läßt jedoch beim Einsatz geringer aufwandmengen in manchen Fällen zu wünschen übrig.

Schließlich wurde schon beschrieben, daß bestimmte in 3-Stellung substituierte Pyridin-Derivate fungizide Eigenschaften besitzen (vgl. US—PS 4 039 675). Auch die Wirksamkeit dieser Verbindungen ist aber nicht immer ausreichend.

Es wurden nun als neue Verbindungen die Phenoxy-pyridinyl-alkanole der Formel (I)

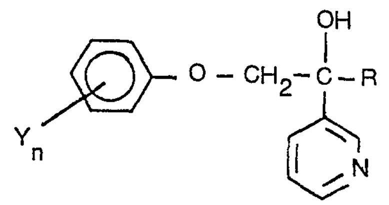

(I)

in welcher

R für Alkyl mit 1 bis 4 Kohlenstoffatomen steht, ferner für Phenyl und Benzyl steht, die gegebenenfalls am Benzolring durch Halogen, Alkyl mit 1 bis 4 Kohlenstoffatomen, Cycloalkyl mit 5 bis 7 Kohlenstoffatomen, Halogenalkyl mit bis zu 2 Kohlenstoff- und bis zu 5 gleichen oder verschiedenen Halogenatomen, durch Alkoxy und Alkylthio mit bis zu 2 Kohlenstoffatomen und durch (weiteres) Phenyl, Phenoxy, Phenylalkoxy oder Phenylalkyl mit jeweils 1 bis 2 Kohlenstoffatomen im Alkylteil substituiert sein können, wobei die aromatischen Zweitsubstituenten noch durch Fluor, Chlor, Brom und/oder Alkyl mit bis zu 4 Kohlenstoffatomen substituiert sein können,

Y für Halogen, Alkyl mit bis zu 4 Kohlenstoffatomen, Cycloalkyl mit 5 bis 7 Kohlenstoffatomen, Halogenalkyl mit bis zu 2 Kohlenstoff- und bis zu 5 gleichen oder verschiedenen Halogenatomen, für Alkoxy und Alkylthio mit bis zu 2 Kohlenstoffatomen, sowie für Phenyl, Phenoxy, Phenalkoxy oder Phenylalkyl mit jeweils 1 oder 2 Kohlenstoffatomen im Alkylteil steht, wobei die genannten aromatischen Reste durch Halogen und/oder Alkyl mit bis zu 4 Kohlenstoffatomen substituiert sein können und

n für ganze Zahlen von 0 bis 3 steht,

sowie deren physiologisch verträgliche Säureadditions-Salze und Komplexe mit den Metallsalzen des Kupfers, Zinks, Mangans, Magnesiums, Zinns, Eisens und Nickels gefunden.

Die erfindungsgemäßen Verbindungen der Formel (I) eignen sich sehr gut als Fungizide auf dem Pflanzensektor.

Die Verbindungen der Formel (I) besitzen ein asymmetrisches Kohlenstoffatom; sie können deshalb in den beiden optischen Isomeren oder als Racemat vorliegen. Sämtliche Isomeren werden erfindungsgemäß beansprucht.

Weiterhin wurde gefunden, daß man die Phenoxy-pyridinylalkanole der Formel (I) sowie deren physiologisch verträgliche Säureadditions-Salze und Komplexe mit Metallsalzen des Kupfers, Zinks, Mangans, Magnesiums, Zinns, Eisens und Nickels erhält, wenn man Phenoxyketone der Formel (I)

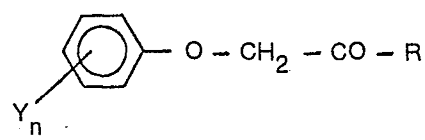

(II)

in welcher

R, Y und n die in Anspruch 1 angegebene Bedeutung haben, mit einem Pyridinyl-halogenid der Formel (III)

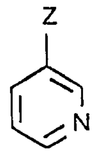

(III)

in welcher
Z für Halogen steht,
in Gegenwart eines Verdünnungsmittels und einer alkalimetall-organischen Verbindung umsetzt und gegebenenfalls anschließend eine Säure oder ein Metallsalz addiert.

Überraschenderweise zeigen die erfindungsgemäßen Phenoxypyridinyl-alkanole eine erheblich höhere fungizide Wirksamkeit, insbesondere gegen Mehltauarten, als die aus dem Stand der Technik bekannten Trityl-1,2,4-triazole, wie Triphenyl-(1,2,4-trizazol-1-yl)-methan, und als Zinkethylen-1,2-bis-dithiocarbamidat, welche bekannte Stoffe gleicher Wirkungsrichtung sind. Außerdem übertreffen die erfindungsgemäßen Stoffe auch die konstitutionell ähnlichsten vorbekannten 5-Pyrimidinyl-carbinole und in 3-Stellung substituierte Pyridin-Derivate bezüglich ihrer fungiziden Eigenschaften auf dem Pflanzensektor. Eine derartige Überlegenheit konnte aufgrund des bekannten Standes der Technik nicht erwartet werden.

Verwendet man 1-(4-Chlorphenoxy)-3,3-dimethyl-butan-2-on und 3-Brompyridin in Gegenwart von n-Butyl-lithium als Ausgangsstoffe, so kann der Reaktionsablauf durch das folgende Formelschema wiedergegeben werden:

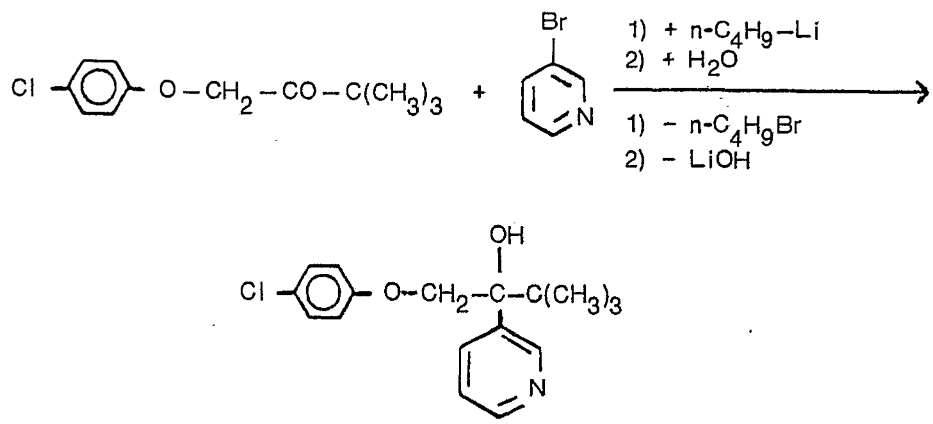


Die als Ausgangsstoffe zu verwendenden Phenoxyketone sind durch die Formel (II) allgemein definiert. Diese Stoffe sind bekannt (vgl. DT—OS 2 105 490 und DT—OS 2 201 063) oder lassen sich nach bekannten Verfahren leicht herstellen. Man erhält sie zum Beispiel, indem man entsprechende Phenole mit entsprechenden Halogenketonen in Gegenwart eines Säurebinders und eines Verdünnungsmittels umsetzt.

Die außerdem als Ausgangsstoffe zu verwendenden Pyridinyl-halogenide sind durch die Formel (III) allgemein definiert. In dieser Formel steht Z vorzugsweise für Chlor und Brom.

Die Halogenide der Formel (III) sind allgemein bekannte Verbindungen der organischen Chemie.

Für die erfindungsgemäße Umsetzung kommen als Verdünnungsmittel vorzugsweise inerte organische Lösungsmittel in Frage. Hierzu gehören vorzugsweise solche, die einen tiefen Festpunkt besitzen, wie insbesondere Ether, wie Diethylether oder Tetrahydrofuran. Vorzugsweise arbeitet man mit Mischungen aus diesen beiden Ethern.

Als alkalimetall-organische Verbindungen werden bei der erfindungsgemäßen Umsetzung vorzugsweise Alkalimetall-alkyle, wie insbesondere n-Butyl-lithium, eingesetzt, es können aber auch Alkalimetall-aryle, wie Phenyl-lithium, Verwendung finden.

Die Reaktionstemperaturen können beim erfindungsgemäßen Verfahren in einem bestimmten Bereich variiert werden. Im allgemeinen arbeitet man zwischen —150 und —50°C, vorzugsweise zwischen —120 und —80°C.

Die erfindungsgemäße Umsetzung wird vorzugsweise unter Inertgas, wie insbesondere Stickstoff oder Argon, vorgenommen.

Bei der Durchführung des erfindungsgemäßen Verfahrens setzt man die Phenoxiketone der Formel (II) und die Halogenide der Formel (III) in etwa äquimolaren Mengen ein, Unter- oder Überschreitungen um bis zu ca. 20 Molprozent sind jedoch möglich. Die alkalimetall-organische Verbindung wird vorteilhaft im Überschuß von 5 bis 75 Molprozent, vorzugsweise von 10 bis 50 Molprozent, verwendet. Es kann dabei so verfahren werden, daß man zunächst die alkalimetall-organische Verbindung mit dem Halogenid der Formel (III) reagieren läßt und anschließend die Keto-Verbindung der Formel (II) zufügt; man kann aber auch die Keto-Verbindung und das Halogenid vorlegen und anschließend bei tiefer

Temperatur (z.B. bei —100 bis —130°C) die alkalimetall-organische Verbindung zugeben.

Die Isolierung der Verbindungen der Formel (I) erfolgt in der Weise, daß das bei der Reaktion zunächst gebildete Alkalialkanolat (z.B. Lithium-alkanolat) mit Wasser hydrolisiert wird. Die weitere Aufarbeitung erfolgt dann in üblicher Weise.

Zur Herstellung von Säureadditionssalzen der Verbindungen der Formel (I) kommen alle physiologisch verträglichen Säuren infrage. Hierzu gehören vorzugsweise die Halogenwasserstoff-säuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen Salze des Kupfers, Zinks, Mangans, Magnesiums, Zinns, Eisens und des Nickels in Frage. Als Anionen der Salze kommen solche in Betracht, die sich von physiologisch verträglichen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z.B. durch Lösen des Metallsalzes in Alkohol, z.B. Aethanol, und Hinzufügen zur Verbindung der Formel (I). Man kann Metallsalzkomplexe in bekannter Weise, z.B. durch Abfiltrieren, isolieren und gegebenenfalls durch Umkristallisation reinigen.

Die erfindungsgemäßen Wirkstoffe weisen eine starke fungitoxische Wirkung auf dem Pflanzensektor auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet, Fungitoxische Mittel im Pflanzenschutz werden eingesetzt zur Bekämpfung von Plasmodiophoromycetes, Oomycetes, Chytridiomycetes, Zygomycetes, Asomycetes, Basidiomycetes, Deuteromycetes.

Die erfindungsgemäßen Wirkstoffe haben eine breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung von echten Mehltaupilzen, beispielsweise zur Bekämpfung von Apfelmehltau (Podosphaera leucotricha) und Getreidemehltau sowie gegen andere Getreidekrankheiten verwendet werden; außerdem insbesondere zur Bekämpfung der Pilze Pyricularia und Pellicularia.

Besonders hervorzuheben ist die teilweise systemische Wirkung der Stoffe. So gelingt es, Pflanzen gegen Pilzbefall zu schützen, wenn man den Wirkstoff über den Boden und die Wurzel den oberirdischen Teilen der Pflanze zuführt.

Als Pflanzenschutzmittel können die erfindungsgemäßen Wirkstoffe zur Saatgut- oder Bodenbehandlung und zur Behandlung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können also solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen Spritzen, Sprühen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentrationen in den Anwendungsformen in einem größeren Bereich variiert werden. Sie liegen im allgemeinen zwischen 0,1 und 0,00001 Gewichtsprozenten, vorzugsweise zwischen 0,05 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Zur Bodenbehandlung sind Wirkstoffmengen von 1 bis 1000 g je cbm Boden, wie insbesondere 10 bis 200 g, erforderlich.

Die vielseitigen Verwendungsmöglichkeiten gehen aus den nachfolgenden Beispielen hervor.

## Beispiel A

Gerstenmehltau-Test (Erysiphe graminis var. hordei)/systemisch
(pilzliche Getreidesproßkrankheit)

Die Anwendung der Wirkstoffe erfolgt als pulverförmige Saatgutbehandlungsmittel. Sie werden hergestellt durch Abstrecken des jeweiligen Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3x12 Korn in Blumentöpfe 2 cm tief in ein Gemisch aus einem Volumteil Fruhstorfer Einheitserde und einem Volumteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21 bis 22°C und 80 bis 90% rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Wirkstoffe, Wirkstoffkonzentrationen im Saatgutbehandlungsmittel sowie dessen Aufwandmenge und der prozentuale Mehltaubefall werden ermittelt. In diesem Test zeigt z.B. folgende Verbindung eine sehr gute Wirkung, die derjenigen der aus dem Stande der Technik bekannten Verbindungen deutlich überlegen ist:

Verbindung gemäß Herstellungsbeispiel 2.

## Beispiel B

Sproßbehandlungs-Test/Getreidemehltau/protektiv
(blattzerstörende Mykose)

Zur Herstellung einer zweckmäßigen Wirkstoffzubereitung nimmt man 0,25 Gewichtsteile Wirkstoff in 25 Gewichtsteilen Di-methylformamid und 0,06 Gewichtsteilen Alkylaryl-polyglykoläther auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21 bis 22°C und einer Luftfeuchtigkeit von 80 bis 90% wertet man den Besatz der Pflanzen mit Mehltaupusteln aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Mehltaubefall ist.

Wirkstoffe, Wirkstoffkonzentrationen in der Spritzbrühe und Befallsgrade werden ermittelt. In diesem Test zeigen z.B. folgende Verbindungen eine sehr gute Wirkung, die derjenigen der aus dem Stande der Technik bekannten Verbindungen deutlich überlegen ist:

Verbindungen gemäß Herstellungsbeispielen 2, 8, 3.

## Beispiel C

Podosphaera-Test (Apfel)/Protektiv

| | | |
|---|---|---|
| Lösungsmittel: | 4,7 | Gewichtsteile Aceton |
| Emulgator: | 0,3 | Gewichtsteile Alkyl-arylpolyglykoläther |
| Wasser: | 95,0 | Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschließend werden sie durch Bestäuben mit Konidien des Apfelmehltauerregers (Podosphaera leucotricha) inokuliert und in ein Gewächshaus mit einer Temperatur von 21 bis 23°C und einer relativen Luftfeuchtigkeit von ca. 70% gebracht.

10 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt. In diesem Test zeigen z.B. folgende Verbindungen eine sehr gute Wirkung, die derjenigen der aus dem Stande der Technik bekannten Verbindungen deutlich überlegen ist:

Verbindungen gemäß Herstellungsbeispielen 2, 3, 8.

## Beispiel D

Myzelwachstums-Test

Verwendeter Nährboden:

| | |
|---|---|
| 20 | Gewichtsteile Agar-Agar |
| 200 | Gewichtsteile Kartoffeldekokt |
| 5 | Gewichtsteile Malz |
| 15 | Gewichtsteile Dextrose |
| 5 | Gewichtsteile Pepton |
| 2 | Gewichtsteile Dinatriumhydrogenphosphat |
| 0,3 | Gewichtsteile Calciumnitrat |

Verhältnis von Lösungsmittelgemisch zum Nährboden:

| | |
|---|---|
| 2 | Gewichtsteile Lösungsmittelgemisch |
| 100 | Gewichtsteile Agarnährboden |

Zusammensetzung Lössungsmittelgemisch

| | |
|---|---|
| 0,19 | Gewichtsteile Dimethylformamid oder Aceton |
| 0,01 | Gewichtsteile Emulgator Alkylaryl-polyglykoläther |
| 1,80 | Gewichtsteile Wasser |
| 2 | Gewichtsteile Lösungsmittelgemisch |

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten beimpft und bei etwa 21 °C inkubiert.

Die Auswertung erfolgt je nach der Wachstumsgeschwindigkeit der Pilze nach 4—6 Tagen. Bei der Auswertung wird das radiale Myzelwachstum auf den behandelten Nährböden mit dem Wachstum auf dem Kontrollnährboden verglichen. Die Bonitierung des Pilzwachstums geschieht mit folgenden Kennzahlen:

1 kein Pilzwachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Wirkstoffe, Wirkstoffkonzentrationen und Resultate werden ermittelt. Dabei zeigen z.B. folgende Verbindungen eine sehr gute Wirkung, die derjenigen der aus dem Stand der Technik bekannten Verbindungen deutlich überlegen ist:

Verbindungen gemäß Herstellungsbeispielen 2, 3, 8.

## Beispiel E

Pyricularia- und Pellicularia-Test

| | |
|---|---|
| Lösungsmittel: | 11,75 Gewichtsteile Aceton |
| Dispergiermittel: | 0,75 Gewichtsteile Alkyl-aryl-polyglykoläther |
| Wasser: | 987,50 Gewichtsteile |
| andere Zusätze: | — Gewichtsteile — |

Man vermischt die für die gewünschte Wirkstoffkonzentration in der Spritzflüssigkeit nötige Wirkstoffmenge mit der angegebenen Menge des Lösungsmittels und des Dispergiermittels und verdünnt das Konzentrat mit der angegebenen Menge Wasser.

Mit der Spritzflüssigkeit bespritzt man etwa 2 bis 4 Wochen alte Reispflanzen bis zur Tropfnässe. Die Pflanzen verbleiben bis zum Abtrocknen in einem Gewächshaus bei Temperaturen von 22 bis 24°C und einer relativen Luftfeuchtigkeit von etwa 70%.

Danach wird der eine Teil der Pflanzen mit einer wässrigen Suspension von 100 000 bis 200 000 Sporen/ml von Pyricularia oryzae inokuliert und in einem Raum bei 24 bis 26°C und 100% relativer Luftfeuchtigkeit aufgestellt. Der andere Teil der Pflanzen wird mit einer auf Malzagar gezogenen Kultur von Pellicularia sasakii infiziert und bei 28 bis 30°C sowie 100% relativer Luftfeuchtigkeit aufgestellt.

5 bis 8 Tage nach der Inokulation wird der Befall bei allen zur Zeit der Inokulation mit Pyricularia oryzae vorhandenen Blättern in Prozent der unbehandelten, aber ebenfalls inokulierten Kontrollpflanzen bestimmt. Bei den mit Pellicularia sasakii infizierten Pflanzen wird der Befall nach der gleichen Zeit an den Blattscheiden ebenfalls im Verhältnis zur unbehandelten, aber infizierten Kontrolle bestimmt. Die Auswertung erfolgt in Wertzahlen von 1—9. 1 bedeutet 100%ige Wirkung, 3 — gute Wirkung, 5 — mäßige Wirkung und 9 — keine Wirkung.

Wirkstoffe, Wirkstoffkonzentrationen und Resultate werden ermittelt. Dabei zeigen z.B. folgende Verbindungen eine sehr gute Wirkung, die derjenigen der aus dem Stande der Technik bekannten Verbindungen deutlich überlegen ist:

Verbindungen gemäß Herstellungsbeispiele 2, 3, 8.

## Herstellungsbeispiele

### Beispiel 1

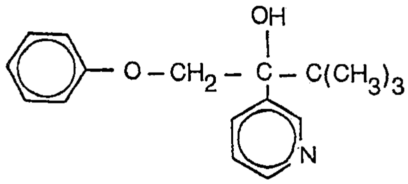

Unter trockener Stickstoffatmosphäre werden zu einer auf —110°C gekühlten Lösung von 12,6 g (0,1 Mol) 3-Brompyridin in 150 ml einer 2:1 Mischung aus absolutem Tetrahydrofuran und absolutem Äther 50 ml einer 15%igen Lösung von n-Butyllithium (enthaltend etwa 0,12 Mol) in n-Hexan langsam zugetropft. Nach beendeter Zugabe läßt man die Reaktionsmischung auf —80°C erwärmen, rührt bei dieser Temperatur 10 Minuten nach und kühlt anschließend die Mischung auf —110 bis —120°C hinunter. Bei dieser Temperatur wird eine Lösung von 19,2 g (0,1 Mol) 3,3-Dimethyl-1-phenoxy-butan-2-on in 80 ml absolutem Tetrahydrofuran zugetropft. Danach läßt man bei –78°C über Nacht rühren. Hiernach läßt man auf Raumtemperatur erwärmen und fügt 200 ml Äther hinzu.

Die Reaktionsmischung wird dreimal mit ln-Salzsäure extrahiert. Die vereinigten Salzsäure-Extrakte werden mit Äther gewaschen, auf festes Natriumbicarbonat gegossen und mehrmals mit Essigester extrahiert. Die Essigesterlösung wird mit Wasser gewaschen, über Natriumsulfat getrocknet und eingeengt. Der Rückstand wird aus Cyclohexan umkristallisiert. Man erhält 15 g (55% der Theorie) 3,3-Dimethyl-1-phenoxy-2-pyridin-3-yl-butan-2-ol vom Schmelzpunkt 89—90,5°C.

Analog werden die Verbindungen der allgemeinen Formel (I)

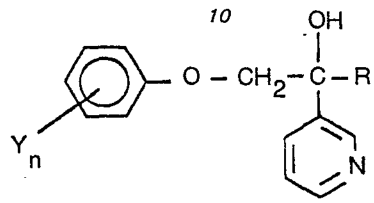

(I)

erhalten:

| Bsp. Nr. | $Y_n$ | R | Schmelzpunkt (°C) |
|---|---|---|---|
| 2 | 2—$CH_3$ | $C(CH_3)_3$ | 66—68 |
| 3 | 2,4—$Cl_2$ | $C(CH_3)_3$ | 113,5—115 |
| 4 | 4—Br | $C(CH_3)_3$ | 171—173 |
| 5 | 4—$OCH_3$ | $C(CH_3)_3$ | 178—180 |
| 6 | 4—Cl | $C(CH_3)_3$ | 162—164 |
| 7 | 4—$CH_3$ | $C(CH_3)_3$ | 131—133 |
| 8 | 2—$CH_3$,4—Cl | $C(CH_3)_3$ | 127—128 |
| 9 | 4—F | $C(CH_3)_3$ | 120—121 |
| 10 | 3,4—$Cl_2$ | $C(CH_3)_3$ | 125—126,5 |
| 11 | 3—$CF_3$ | $C(CH_3)_3$ | 132—133,5 |
| 12 | 4—O—$CH_2$— 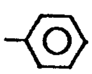 | $C(CH_3)_3$ | 125—126 |
| 13 | 2—$OCH_3$,4—Cl | $C(CH_3)_3$ | 128—129 |
| 14 | 4— 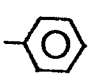 | $C(CH_3)_3$ | 120—121 |
| 15 | 2,4—$Cl_2$ | 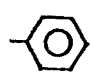 | >238(Zers.) (x½NDS) |
| 15 | — | 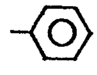 | 194—197(x½NDS) |
| 17 | 4—Cl | 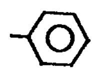 | 182—185(x½NDS) |
| | | 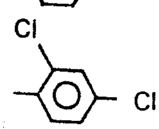 | |
| 18 | 4—Cl | 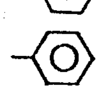 | >250(x½NDS) |
| 19 | 2—$CH_3$,4—Cl | | 187—190(x½NDS) |

| Bsp. Nr. | $Y_n$ | R | Schmelzpunkt (°C) |
|---|---|---|---|
| 20 | 3—$CF_3$ | 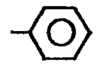 | 238—240($x\frac{1}{2}$NDS) |
| 21 | 4—$OCH_3$ | 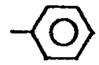 | 175—177($x\frac{1}{2}$NDS) |
| 22 | 2,4—$Cl_2$ | $CH_3$ | 98,5—100 |
| 23 | — | $CH_3$ | 204—208($x\frac{1}{2}$NDS) |
| 24 | 4—$CH_3$ | $CH_3$ | 184—186($x\frac{1}{2}$NDS) |
| 25 | 4—$OCH_3$ | $CH_3$ | 200—203($x\frac{1}{2}$NDS) |
| 26 | 4—O- 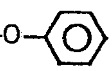 | $C(CH_3)_3$ | 103—104°C |
| 27 | 4—F | 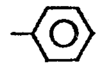 | 222—225°C($x\frac{1}{2}$NDS) |
| 28 | 2,4—$Cl_2$ | 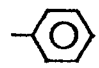 | zähfl. Öl |

NDS = 1,5-Naphthalindisulfonsäure

**Patentansprüche**

1. Phenoxy-pyridinyl-alkanole der Formel (I)

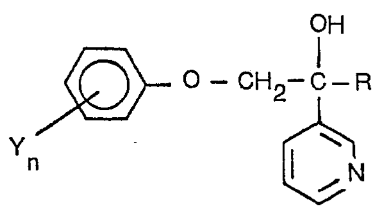

(I)

in welcher
R für Alkyl mit 1 bis 4 Kohlenstoffatomen steht, ferner für Phenyl und Benzyl steht, die gegebenenfalls am Benzolring durch Halogen, Alkyl mit 1 bis 4 Kohlenstoffatomen, Cycloalkyl mit 5 bis 7 Kohlenstoffatomen, Halogenalkyl mit bis zu 2 Kohlenstoffund bis zu 5 gleichen oder verschiedenen Halogenatomen, durch Alkoxy und Alkylthio mit bis zu 2 Kohlenstoffatomen und durch (weiteres) Phenyl, Phenoxy, Phenylalkoxy oder Phenylalkyl mit jeweils 1 bis 2 Kohlenstoffatomen im Alkylteil substituiert sein können, wobei die aromatischen Zweitsubstituenten noch durch Fluor, Chlor, Brom und/oder Alkyl mit bis zu 4 Kohlenstoffatomen substituiert sein können,
Y für Halogen, Alkyl mit bis zu 4 Kohlenstoffatomen, Cycloalkyl mit 5 bis 7 Kohlenstoffatomen, Halogenalkyl mit bis zu 2 Kohlenstoff- und bis zu 5 gleichen oder verschiedenen Halogenatomen, für Alkoxy und Alkylthio mit bis zu 2 Kohlenstoffatomen, sowie für Phenyl, Phenoxy, Phenalkoxy oder Phenylalkyl mit jeweils 1 oder 2 Kohlenstoffatomen im Alkylteil steht, wobei die genannten aromatischen Reste durch Halogen und/oder Alkyl mit bis zu 4 Kohlenstoffatomen substituiert sein können und
n für ganze Zahlen von 0 bis 3 steht,
sowie deren physiologisch verträgliche Säureadditions-Salze und Komplexe mit den Metallsalzen des Kupfers, Zinks, Mangans, Magnesiums, Zinns, Eisens und Nickels.

2. Verfahren zur Herstellung von Phenoxy-pyridinyl-alkanolen gemäß Anspruch 1, dadurch gekennzeichnet, daß man Phenoxyketone der Formel (II)

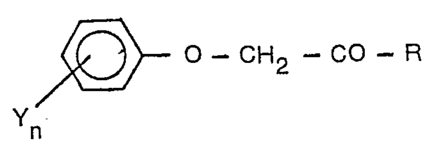

(II)

in welcher

R, Y und n die in Anspruch 1 angegebene Bedeutung haben, mit einem Pyridinyl-halogenid der Formel (III)

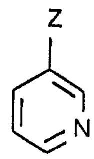

(III)

in welcher
Z für Halogen steht,
in Gegenwart eines Verdünnungsmittels und einer alkalimetall-organischen Verbindung umsetzt und gegebenenfalls anshließend eine Säure oder ein Metallsalz addiert.

3. Fungizide Mittel, gekennzeichnet durch einen Gehalt an mindestens einem Phenoxy-pyridinyl-alkanol gemäß Anspruch 1.

4. Verwendung von Phenoxy-pyridinyl-alkanolen gemäß Anspruch 1 zur Bekämpfung von Pilzen auf dem Pflanzensektor.

5. Verfahren zur Herstellung von fungiziden Mitteln, dadurch gekennzeichnet, daß man Phenoxy-pyridinylalkanole gemäß Anspruch 1 mit Streckmitteln und/oder oberflächenaktiven Mitteln vermischt.

**Revendications**

1. Phénoxy-pyridinyl-alcanols de formule (I):

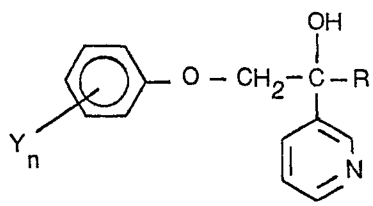

(I)

dans laquelle
R représente un groupe alkyle contenant 1 à 4 atomes de carbone, de même qu'un groupe phényle ou benzyle pouvant éventuellement être substitué sur le noyau benzène par un atome d'halogène, par un groupe alkyle contenant 1 à 4 atomes de carbone, par un groupe cycloalkyle contenant 5 à 7 atomes de carbone, par un groupe halogénalkyle contenant jusqu'à 2 atomes de carbone, et jusqu'à 5 atomes d'halogènes identiques ou différents, par un groupe alcoxy ou par un groupe alkylthio contenant jusqu'à 2 atomes de carbone et également par un groupe phénylalkyle, phénylalcoxy, phénoxy ou phényle (supplémentaire) contenant chacun 1 à 2 atomes de carbone dans la fraction alkyle, les seconds substituants aromatiques pouvant encore être substitués par un atome de fluor, un atome de chlore, un atome de brome et/ou un groupe alkyle contenant jusqu'à 4 atomes de carbone,
Y représente un atome d'halogène, un groupe alkyle contenant jusqu'à 4 atomes de carbone, un groupe cycloalkyle contenant 5 à 7 atomes de carbone, un groupe halogénalkyle contenant jusqu'à 2 atomes de carbone et jusqu'à 5 atomes d'halogènes identiques ou différents, un groupe alcoxy et un groupe alkylthio contenant jusqu'à 2 atomes de carbone, de même qu'un groupe phényle, phénoxy, phénalcoxy ou phénylalkyle contenant chacun 1 ou 2 atomes de carbone dans la fraction alkyle, les radicaux aromatiques mentionnés pouvant être substitués par un atome d'halogène et/ou par un groupe alkyle contenant jusqu'à 4 atomes de carbone, et
n représente un nombre entier de 0 à 3,
de même que leurs sels d'addition d'acide et leurs complexes (physiologiquement compatibles) avec les sels métalliques du cuivre, du zinc, du manganèse, du magnésium, de l'étain, du fer et du nickel.

2. Procédé de préparation de phénoxy-pyridinyl-alcanols suivant la revendication 1, caractérisé en ce qu'on fait réagir des phénoxycétones de formule (II):

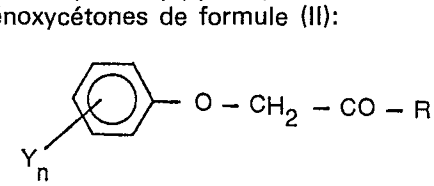

(II)

dans laquelle
R, Y et n ont les significations indiquées dans la revendication 1,
avec un halogénure de pyridinyle de formule (III):

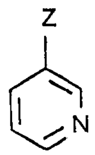

(III)

dans laquelle

Z représente un atome d'halogène,

en présence d'un diluant et d'un composé organique d'un métal alcalin, puis on ajoute éventuellement un acide ou un sel métallique.

3. Agents fongicides, caractérisés en ce qu'ils contiennent au moins un phénoxy-pyridinyl-alcanol suivant la revendication 1.

4. Utilisation de phénoxy-pyridinyl-alcanols suivant la revendication 1 pour combattre les champignons dans le domaine des plantes.

5. Procédé de préparation d'agents fongicides, caractérisé en ce qu'on mélange des phénoxy-pyridinyl-alcanols suivant la revendication 1 avec des diluants et/ou des agents tensio-actifs.

**Claims**

1. Phenoxy-pyridinyl-alkanols of the formula (I)

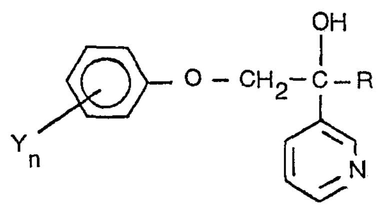

(I)

in which

R represents alkyl with 1 to 4 carbon atoms, and further represents phenyl and benzyl, which may optionally be substituted in the benzene ring by halogen, alkyl with 1 to 4 carbon atoms, cycloalkyl with 5 to 7 carbon atoms, halogenoalkyl with up to 2 carbon atoms and up to 5 identical or different halogen atoms, by alkoxy and alkylthio with up to 2 carbon atoms and by (further) phenyl, phenoxy, phenylalkoxy or phenylalkyl with in each case 1 to 2 carbon atoms in the alkyl part, it being possible for the aromatic secondary substituents to be also substituted by fluorine, chlorine, bromine and/or alkyl with up to 4 carbon atoms,

Y represents halogen, alkyl with up to 4 carbon atoms, cycloalkyl with 5 to 7 carbon atoms, halogenoalkyl with up to 2 carbon atoms and up to 5 identical or different halogen atoms, alkoxy and alkylthio with up to 2 carbon atoms, as well as phenyl, phenoxy, phenalkoxy or phenylalkyl with in each case 1 or 2 carbon atoms in the alkyl part, it being possible for the indicated aromatic radicals to be substituted by halogen and/or alkyl with up to 4 carbon atoms and

n represents integers from 0 to 3,

and their physiologically acceptable acid addition salts and complexes with the metal salts of copper, zinc, manganese, magnesium, tin, iron and nickel.

2. Process for the preparation of phenoxy-pyridinylalkanols according to Claim 1, characterised in that phenoxyketones of the formula (II)

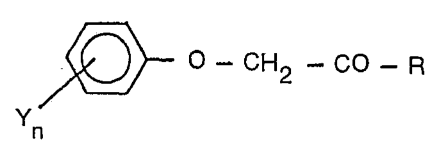

(II)

in which

R, Y and n have the meaning indicated in Claim 1, are reacted with a pyridinyl halide of the formula (III)

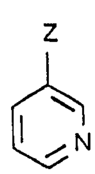

(III)

in which

Z represents halogen,

in the presence of a diluent and an alkali metal-organic compound and, if required, an acid or a metal salt is subsequently added on.

3. Fungicidal compositions, characterised in that they contain at least one phenoxy-pyridinyl-alkanol according to Claim 1.

4. Use of phenoxy-pyridinyl-alkanols according to Claim 1 for combating fungi in the plant sector.

5. Process for the production of fungicidal compositions, characterised in that phenoxy-pyridinyl-alkanols according to Claim 1 are mixed with extenders and/or surface-active agents.